(19) Europäisches Patentamt
European Patent Office
Office européen des brevets

(11) Numéro de publication: **0 372 063 B1**

# (12) FASCICULE DE BREVET EUROPEEN

(45) Date de publication de fascicule du brevet: **28.09.94**

(51) Int. Cl.$^5$: **A61K 37/50**, C12N 15/53, C12N 9/08

(21) Numéro de dépôt: **89907125.2**

(22) Date de dépôt: **13.06.89**

(86) Numéro de dépôt internationale : **PCT/EP89/00668**

(87) Numéro de publication internationale : **WO 89/12457 (28.12.89 89/30)**

## (54) MYELOPEROXYDASE HUMAINE ET APPLICATION THERAPEUTIOUE.

(30) Priorité: **14.06.88 FR 8807914**

(43) Date de publication de la demande: **13.06.90 Bulletin 90/24**

(45) Mention de la délivrance du brevet: **28.09.94 Bulletin 94/39**

(84) Etats contractants désignés: **AT BE CH DE FR GB IT LI LU NL SE**

(56) Documents cités:
**EP-A- 0 098 073**
**GB-A- 2 108 387**

**Proc. Natl. Acad. Sci. USA, vol. 84, avril 1987, S.C. Weil et al., pp. 2057-2061**

**The Journal of Biological Chemistry, vol. 262, no. 8, 15 mars 1987, The American Society of Biological Chemists, Inc. (US), K. Morishita et al., pp. 3844-3851**

(73) Titulaire: **LA REGION WALLONNE**
**Rue des Colonies, 52 (Bte 5)**
**B-1000 Bruxelles (BE)**

(72) Inventeur: **DEBY, Carol**
**37, quai de l'Ourthe**
**B-4020 Liège (BE)**
Inventeur: **PINCEMAIL, Joel**
**48, rue du Canal**
**B-4050 Hony-Esneux (BE)**
Inventeur: **BOLLEN, Alex**
**Gaasbeekstraat 69**
**B-1711 Itterbeek (BE)**

(74) Mandataire: **Warcoin, Jacques et al**
**Cabinet Régimbeau**
**26, avenue Kléber**
**F-75116 Paris (FR)**

EP 0 372 063 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne l'enzyme myéloperoxydase humaine (hMPO) sa préparation par génie génétique et son utilisation à titre de médicament. Elle concerne donc également son utilisation pour la fabrication de compositions pharmaceutiques en contenant ainsi que les compositions pharmaceutiques elles-mêmes.

Plus précisément, l'application thérapeutique visée a pour objet le traitement des immunodéficients en thérapie humaine par le renforcement de l'activité antimicrobienne au niveau des macrophages, et ceci dans tous les cas d'immunodéficiences qu'elles soient occasionnées notamment par le SIDA, les brûlures ou les irradiations. L'enzyme naturelle myéloperoxydase humaine et son utilisation thérapeutique ont été décrites par exemple dans la demande de brevet GB-A-2108387. Le gène codant pour l'enzyme a été isolé et sa séquence nucléotidique élucidée par Kazuhiro Morishita et al. Journal Biolog. Chem., Vol. 262, No. 8, Mars 15, 1987, pages 3844-3851.

La résistance à l'infection par les micro-organismes met en oeuvre des fonctions non-spécifiques (action d'enzymes, pH, paroi épithéliale) et des réponses immunitaires adaptatives des cellules lymphocytes B et T.

Les fonctions non spécifiques empêchent l'invasion de la majorité des agresseurs. Cependant, lors-que cette première ligne de défense cède, le système phagocytaire entre en jeu, détruit les agents infectieux et stimule les fonctions d'immunité conférées par les cellules B et T.

Toute anomalie, héréditaire ou acquise, du système phagocytaire a des conséquences graves puisque même les micro-organismes normalement peu pathogènes y échappent et déclenchent des infections récurrentes.

Par ailleurs, toute déficience du système immunitaire lui-même, au niveau des cellules T ou B, conduit à une susceptibilité exacerbée aux infections virales et bactériennes intra- ou extracellulaires. Ces déficiences peuvent être héréditaires ou acquises (ex : SIDA = déficience élective en cellules T). La plupart des personnes souffrant de ces déficiences subissent l'infection d'organismes opporuunistes (bactéries, protozoaires, etc.).

Dans l'ensemble des cas d'immunodépression, il est donc souhaitable que le système phagocytaire soit aussi efficace que possible pour limiter les conséquences des agressions extérieures. Accessoire en conditions normales, la phagocytose revêt un caractère essentiel lorsque la réponse immune B et T s'affaiblit.

Parmi les cellules associées à la réponse immunitaire, les leucocytes polymorphonucléaires représentent un intérêt particulier dans le contexte de la lutte contre les infections. Ces cellules contiennent une enzyme, la myéloperoxydase, dont l'action microbicide est bien documentée. Les polynucléaires ne présentent aucune spécificité vis-à-vis d'un antigène, mais jouent un rôle essentiel en cas d'inflammation aiguë, avec les anticorps et le système du complément, dans la défense de l'hôte contre les micro-organismes. Leur fonction principale est la phagocytose. Au cours de ce processus, les micro-organismes sont inclus dans des vacuoles (phagosomes) qui fusionnent avec les granules contenant la myéloperoxydase pour former les phagolysosomes. Au cours de la phagocytose, l'activité enzymatique de la myéloperoxydase conduit à la formation d'HOCl, un composé bactéricide (acide hypochloreux) puissant; cette activité requiert $H_2O_2$ (eau oxygénée), qui apparaît dans le polymorphonucléaire lors de sa stimulation par divers agents et notamment par les réactions immunologiques provoquées par les micro-organismes. L'acide hypochloreux est oxydant par lui-même, mais produit des dérivés plus oxydants encore, les chloramines. Enfin, réagissant avec $H_2O_2$, dont il est issu, il produit une forme d'oxygène extrêmement oxydante, l'oxygène singulet.

Le problème majeur se situe toutefois au niveau des macrophages. En effet le macrophage est une très grande cellule, plus robuste que le polymorphonucléaire, capable, à l'instar de ce dernier, de phagocyter les micro-organismes. Il possède également un système générateur d'$H_2O_2$, mais n'est cependant pas capable de produire la myéloperoxydase. Cette déficience diminue son efficacité défensive. On a découvert toutefois selon l'invention, que les macrophages peuvent <u>incorporer</u> et <u>utiliser</u> la myéloperoxydase qui reste active après pénétration dans les macrophages, acquisition complétant efficacement leur arsenal cytolytique et bactériolytique, en particulier pour la destruction d'agents infectieux divers affectant les patients immunodéprimés.

Quoique la myéloperoxydase une fois dans le plasma soit très vite captée par les macrophages, des systèmes d'administration spécifiques délivrant de façon optimale l'enzyme dans les macrophages, peuvent être utilisés selon l'invention, réalisant des conjugués de la myéloperoxydase par couplage covalent avec un transporteur présentant une affinité pour les macrophages. On peut citer à ce titre des transporteurs tels que l'albumine humaine mannosylée mais aussi des anticorps ou fragments d'anticorps tels que la partie

constante Fc dirigés contre des récepteurs présents sur les macrophages.

D'autres systèmes consistent à coupler par complexation non covalente ou par manipulation d'ADN un anticorps ou fragment d'Ac spécifique du macrophage à l'enzyme myéloperoxydase humaine pour obtenir un"complexe immun".

L'administration de tels conjugués ou complexes immuns conduit au ciblage de la MPO humaine vers le macrophage, à son ingestion par phagocytose et à la libération de l'enzyme sous forme active à l'intérieur du macrophage, son site d'action privilégié, où elle participe à la lutte contre les infections.

Dans le cas des complexes immuns préparés par génie génétique, l'ADN codant pour la MPO active ou sous forme de précurseur naturel est couplé à l'ADN codant pour un fragment d'immunoglobuline reconnaissant spécifiquement les macrophages.

La présente invention a donc pour objet, à titre de médicament, un composé consistant dans l'enzyme myéloperoxydase humaine.

L'invention a également pour objet, à titre de médicament, un composé consistant dans un conjugué de la myéloperoxydase par couplage covalent ou complexation avec un transporteur présentant une affinité pour les macrophages, tels que l'albumine humaine mannosylée ou un anticorps ou fragments d'anticorps comme la partie constante Fc dirigée contre des récepteurs présents sur les macrophages.

Un autre composé, objet de la présente invention, délivrant la myéloperoxydase de façon optimale vers les macrophages, consiste également dans des liposomes, notamment des liposomes biopolymérisés, dans lesquels la myéloperoxydase se trouve encapsulée.

Les médicaments selon l'invention sont en particulier utiles pour lutter contre les infections à l'intérieur des macrophages.

L'enzyme myéloperoxydase humaine, utilisée selon l'invention, est d'origine recombinante, c'est-à-dire préparée par génie génétique.

En outre, la présente invention a pour objet l'utilisation des composés selon l'invention pour la fabrication de compositions pharmaceutiques utiles notamment pour le traitement des immunodéficiences occasionnées en particulier par le SIDA, les brûlures ou les irradiations.

Enfin, la présente invention a pour objet des compositions pharmaceutiques comprenant, à titre de principe actif, les composés selon l'invention.

Les compositions pharmaceutiques selon l'invention peuvent se présenter sous différentes formes appropriées pour des formes d'administration diverses notamment par voie parentérale, systémique ou topique ou par injection intra-veineuse dans la mesure où les macrophages cibles sont présents non seulement dans le sang mais aussi dans les autres zones de l'organisme.

Les compositions pharmaceutiques selon l'invention comportent alors outre le principe actif, des supports pharmaceutiques acceptables pour la forme d'administration en cause.

L'administration parentérale de la myéloperoxydase ou de composés selon l'invention est très appropriée pour les divers syndromes immunodépressifs.

La diminution ou la suppression des barrières naturelles et immunitaires favorise l'apparition d'infections dues à des germes pathogènes ou opportunistes.

Les déficiences du système immunitaire (par exemple déficiences en cellules T, SIDA, irradiation, chimiothérapie anticancéreuse...) sont souvent associées à des infections généralisées (bactéries, mycoses, viroses, protozoaires ...) très difficiles à contrôler avec les seuls agents antibiotiques et chimiothérapeutiques existants. Dans ces conditions, la myéloperoxydase peut aussi être employée par voie systémique afin de renforcer l'activité antiseptique des macrophages. au cours de la phagocytose. En effet, les résultats expérimentaux indiquent que les macrophages et les monocytes ont une activité cytolytique et bactériolytique accrue en présence de l'enzyme.

La myéloperoxydase, ou un composé selon l'invention, peut également être administré(e) par application topique sur notamment des ulcères torpides, où la microvascularisation est déficiente (ulcère variqueux).

Pour cette application topique la myéloperoxydase sera incorporée par exemple dans de la pâte à l'eau selon une formulation dermatologique connue. Au moment de l'emploi on pourra procéder à un mélange extemporané de la suspension de myéloperoxydase avec

- soit une solution diluée de peroxyde d'hydrogène (concentration 0,3 % par exemple)
- soit une autre enzyme telle que la xanthine oxydase.

Dans ce cas il convient d'ajouter à la suspension de myéloperoxydase une concentration $10^{-3}$M d'hypoxanthine et éventuellement de chlorure d'ammonium. Ce mélange de pâtes produit un dégagement de HClO et une formation de chlorure d'amine, particulièrement efficace pour le nettoyage des plaies torpides.

D'autres indications thérapeutiques sont envisageables par voie topique :

1. L'utilisation de [a myéloperoxydase est intéressante dans la prévention et le traitement des infections intercurrentes au cours des brûlures thermiques, chimiques ou par irradiation.

2. La fonction phagocytaire apparaît déficitaire au cours de l'eczéma atopique. Dans ce cas, l'application locale de l'enzyme peut avoir une action stimulante bénéfique sur les macrophages et les monocytes cutanés.

3. La myéloperoxydase peut avoir un rôle adjuvant dans le traitement des infections gingivales, que ce soit chez les sujets immunodéprimés ou au cours des parodontoses.

Selon l'invention la myéloperoxydase peut être obtenue par purification analytique à partir de leucocytes polymorphonucléaires. Néanmoins avantageusement l'enzyme myéloperoxydase humaine utilisée sera produite par génie génétique avec la technologie de l'ADN recombinant.

Pour ce faire, les étapes essentielles sont :

1) construction d'un banque de clones d'ADNc représentative des produits synthétisés dans les cellules leucocytaires et criblage de cette banque avec une sonde appropriée caractéristique de la myéloperoxydase humaine. A l'issu de cette opération, on obtient un clone ADNc codant pour l'enzyme.

2) l'ADNc myéloperoxydase doit alors être manipulé pour permettre son expression dans divers système hôtes/vecteurs. Une construction modulaire s'impose si l'on veut être capable d'évaluer l'expression dans les systèmes aussi variés que E. coli, la levure, les cellules de mammifères ou les cellules d'insectes. L'obtention d'une enzyme active, correctement assemblée et processée, est fait avantageusement dans des systèmes de cellules eucaryotes.

La présente invention a donc également pour objet la hMPO produite par culture de cellules de prokaryotes ou d'eukaryotes transformées par un vecteur d'expression de la hMPO dans lesdites cellules.

Avantageusement, la hMPO selon l'invention est produite par cultures de cellules d'eukaryotes supérieurs notamment par des cellules d'insectes ou de mammifères.

Pour ce faire, la présente invention a pour objet :

1) une cassette d'expression HindIII/SnaBI, HindIII/EcorV ou HindIII/HpaI de 2261 pb portant la séquence codante pour la hMPO telles que représentées sur la figure 1, ainsi que le plasmide pNIV2702 contenant lesdites cassettes,

2) un vecteur d'expression dans des cellules de prokaryotes ou de d'eukaryotes comportant ladite cassette, et en particulier

3) un plasmide de transfert recombinant pour Baculovirus contenant l'ADNc de hMPO sous le contrôle du promoteur de polyhédrine, notamment

4) le plasmide pNIV2704 de la figure 5,

5) les cellules d'insectes, notamment de spodoptera frugiperda tels que les cellules Sf9 co-transfectées par un vecteur selon l'invention avec de l'ADN viral sauvage et notamment co-transfecfectées par le plasmide pNIV2704,

6) des cellules d'insectes, notamment de spodoptera frugiperda tels que Sf9 infectées par un Baculovirus recombinant obtenu par un vecteur selon l'invention, notamment à partir du plasmide pNIV2704,

7) la hMPO produite par culture de cellules d'insectes notamment de spodoptera frugiperda tels que Sf9 modifiées par un vecteur d'expression dans lesdites cellules selon l'invention,

8) un vecteur d'expression dans des cellules de mammifères, notamment des cellules CHO contenant la cassette HindIII/SnaBI de la figure 1, notamment

9) un plasmide pNIV2703,

10)des cellules de mammifères, notamment des cellules CHO transfectées par un vecteur d'expression dans lesdites cellules selon l'invention, notamment par le plasmide pNIV2703,

11 )la hMPO produite par culture de cellules de mammifères. notamment de CHO transfectées par un vecteur d'expression dans lesdites cellules selon l'invention.

La description détaillée qui va suivre est destinée à illustrer d'autres caractéristiques et avantages de la présente invention.

La figure 1 représente la cassette HindIII/HpaI de 2261 pb contenant les séquences codant pour la myéloperoxydase humaine et cassette HindIII/SnaBI.

La séquence codante commence à l'ATG spécifiant la méthionine N-terminale (Met) et se termine par le codon stop TGA (***).

Les séquences en gras représentent les oligonucléotides synthétiques ajoutés en 5' et en 3' de l'ADNc de la hMPO.

La figure 2 représente la fixation de sérums de lapins en ELISA sur MPO.

La figure 3 représente la fixation de sérums de souris en ELISA sur MPO.

La figure 4 représente une courbe standard de test ELISA de détection de la myéloperoxydase humaine.

La figure 5 représente la construction du vecteur pAcYMI/ MPOII selon l'invention (plasmide de transfert pour Baculovirus).

La figure 6 représente la carte du vecteur Tnd à partir duquel est construit le plasmide pNIV2703 selon l'invention.

Les 3 cassettes dirigent respectivement l'expression des marqueurs de sélection DHFR et $Neo^R$, ainsi que celle d'un ADNc étranger (dans le cas illustré ci-dessus, il s'agit du t-PA). Cette dernière cassette peut être substituée par une cassette codant pour tout ADNc approprié, en tirant parti des sites de restriction uniques flanquant la cassette t-PA.

Abréviations :

| | |
|---|---|
| SV early : | promoteur précoce de SV40 ; |
| 5 : | extensions non codantes en 5'; |
| 3 : | extensions non codantes en 3'; |
| SV : | région de polyadénylation de SV40 ; |
| Rous LTR : | séquence répétée terminale (longue) dérivée du virus du sarcome de Roux ; |
| bGH : | région de polyadénylation du gène de l'hormone de croissance bovine ; |
| Betablopro : | promoteur principal de la $\beta$-globuline murine. |

Exemple 1 : préparation de la hMPO par génie géretique

A. Construction d'une cassette contenant l'entièreté des séquences codant pour la myéloperoxydase humaine flanquée de sites de-restriction absents de l'ADNc de la myéloperoxydase (MPO).

1. But : Obtention d'une cassette *Hind*III/*Hpa*I contenant les sèquences codant pour la MPO humaine et insérable dans différents vecteurs d'expression.

2. Matériel de départ : - le plasmide bactérien pMPO62 contenant un ADNc de la MPO humaine complet (Johnson et al.,1987, Nucleic Acids Research 15. 2013-2026);

- un vecteur de clonage quelconque contenant des sites de restriction adéquats. Notre choix s'est porté sur le plasmide pTNDPC2, un plasmide dérivé du pTND (Connors et al.,1988, DNA 7 , 651-661).Le plasmide pTNDPC2 n'est pas essentiel à l'obtention de la dite cassette; il aurait été parfaitement possible d'utiliser un autre vecteur de clonage contenant les sites de restriction nécessaires tel que le plasmide pJRD184 (Heusterspreute et al., Gene 39 (1985) 299-309).

3). Obtention d'oligonucléotides par synthèse chimique.

- Pour obtenir un site *Hind*III directement en amont de l'ATG de la hMPO, nous avons choisi de synthétiser, grâce à des méthodes chimiques bien connues dans notre domaine, une paire d'oligonucléotides qui une fois rèhybridés entre eux, contiennent. dans le sens 5'- 3' de la fibre codante, un site de restriction *Hind*III, les bases ACC, les séquences codant pour les 11 premiers acides aminés et la 1ère base du triplet du douzième acide aminé de la hMPO. qui est à cheval sur un site *Nsi*I. Ces oligonucléotides sont dénommés MPOIII et MPOIV (schéma 1 ci-apres).
- Pour obtenir un site *Hpa*I en aval du codon stop de la hMPO. une deuxième paire d'oligonucléotides a été synthétisée. Une fois réhybridés. ceux-ci présentent. dans le sens 5'-3' sur la fibre codante. Les deux dernières bases du triplet de l'acide aminé 731. la séquence codant pour les 14 derniers acides aminés de la hMPO. un codon stop (TGA) différent du codon stop naturel de la hMPO et quinze bases contenant un site *EcoR*V, un site *SnaB*I et le complément d'un site *Hpa*I. Ces oligonucléotides sont dénommés NPOI et MPOII (schéma 1 ci-après).

4. Sous-clonages.

Sous-clonage 1

Un fragment *Nsi*I/*Bgl*II de 2053 pb (paires de bases) a été extrait du plasmide pMPO62. Ce fragment et le fragment synthétique *Hind*III/*Nsi*I de 42 pb obtenu par réhybridation des oligonucléotides MPOIII et MPOIV ont été ligués à un fragment *Hind*III/*Bgl*II de 6755 pb du pTNDPC2. Le plasmide pscMPO1 résultant a été introduit dans la souche d'*Escherichia coli* MM294. suivant une méthode bien connue, dans le but de produire le plasmide pscMPO1 en plus grande quantité. Le plasmide pscMPO1 contient ainsi un fragment de 2095 pb allant d'un site *Hind*III à un site *Bgl*II situé approximativement à l'acide aminé 696 de la hMPO.

Sous-clonage 2

Un fragment *Xba*I/*Pst*I de 1825 pb a été extrait du plasmide pMPO62. Ce fragment et le fragment synthétique *Pst*I/*Hpa*I de 62 pb obtenu par réhybridation des oligonucléotides MPOI et MPOII ont été

ligués à un fragment *Xba*I/*Hpa*I de 3283 pb du TNDPC2. Le plasmide pscMPO2 résultant a été introduit dans la souche d'*Escherichia coli* MM294 dans le but de le produire en plus grande quantité.

Le plasmide pscMPO2 contient donc un fragment de 1887 pb allant d'un site *Xba*I débutant par la première base de l'acide aminé 123 de la hMPO à un site *Hpa*I.

5. Construction du plasmide pNIV2702 à partir des pscMPO1 et pscMPO2

Pour finalement obtenir la cassette hMPO *Hind*III *Hpa*I, un fragment *Hind*III/*Xba*I de 374 pb contenant la séquence codant pour les acides aminés 1 à 122 de la hMPO et un fragment *Xba*I/*Hpa*I de 1887 pb contenant la séquence codant pour les acides aminés 123 à 745 de la hMPO ont été extraits respectivement des plasmides pscMPO1 et pscMPO2. Ces deux fragments ont ensuite été religués à un fragment *Hind*III/*Hpa*I de 5165 pb du TNDPC2 pour obtenir le plasmide pNIV2702.

Le plasmide pNIV2702 contient donc une cassette *Hind*III/*Hpa*I de 2261 pb portant l'entièreté des séquences codant pour la hMPO ( Fig.1). Cette cassette peut aisément être extraite et transférée dans différents vecteurs d'expression pour cellules d'ovaires de hamster (CHO) ou pour cellules d'insectes (*Spodoptera frugiperda*) via le baculovirus.

MPOI

stop

GTACACTTCCTGCATTGAACCTGGCTTCCTGGAGGGAAGCCTCCTGATATCTACGTATGGTT 3'

3' ACGTCATGTGAAGGACGTAACTTGGACCGAAGGACCTCCCTTCGGAGGACTATAGATGCATACCAA 5'

MPOII

*Pst*I     *EcoR*V   *SnaB*I   *Hpa*I

3' terminus MPO

MPOIII

Met

5' AGCTTACCATGGGGGTTCCCTTCTTCTCTTCTCTCAGATGCA 3' 42-mer

3' ATGGTACCCCCAAGGGAAGAAGAGAAGAGAGTCT 5' 34-mer

MPOIV

*Hind*III     *Nsi*I

5' terminus MPO

Schéma 1

B. Mise au point d'un test ELISA pour la détection de myéloperoxydase naturelle et recombinante

a) Obtention d'anticorps anti-MPO

Des sérums anti-MPO ont été obtenus chez le lapin et la souris. Ils ont été testés en ELISA sur MPO. (La plaque est coatée avec MPO et saturée à la BSA. On y ajoute les anticorps à tester, puis un conjugué phosphatase alcaline anti-Ig). Dans les deux cas, on obtient un titre d'anticorps anti-MPO au moins 8.000 fois plus élevé dans le sérum immun que dans un sérum normal (Figs. 2 et 3).

b) Mise au point du test

Un test "en sandwich" a été réalisé en utilisant des Ig de lapin anti-MPO (Prosan-Dakopatts A398) et un de nos sérums de souris anti-MPO. La plaque est coatée avec les Ig de lapin à 7.6 gamma/ml dans PBS pH 7.8, une nuit à 4 °C. Elle est saturée par 1% BSA dans PBS pH 7.8 Tween 20 0,05% 1h40 à température ambiante. Les échantillons à tester sont laissés 2h à température ambiante, puis le sérum de souris, dilué 1000 fois dans la solution de saturation, 2h à température ambiante, enfin le conjugué

Fab2 de lapin anti-Ig de souris-phosphatase alcaline (Prosan-Dakopatts D314) dilué 1000 fois dans du tampon TBS (Tris-HCl 0,05M pH 7.5, NaCl 0,15M) contenant 1% BSA et 0,05% Tween 20. Entre chaque étape la plaque est lavée 5 fois avec soit TBS Tween 20 0,05% (avant et après la mise du conjugué), soit PBS Tween 20 (autres étapes). La révélation se fait grâce à une solution de para-nitrophényl phosphate 1 mg/ml dans 10% diéthanolamine, 0,01% $MgCl_2$ $6H_2O$, 0,02% $NaN_3$ pH 9.8, et l'arrêt de la réaction par NaOH 3M. La lecture se fait à 410 nm. La figure 4 montre une courbe de fixation d'une MPO pure (Green Cross Corporation) diluée dans un Surnageant de culture de cellules d'insecte SFMJ (milieu TC100 avec 10% FCS). On voit que le test est utilisable pour doser la MPO dans une gamme de concentrations comprises entre 0,1 ng/ml et 100 ng/ml.

C) Clonage de l'ADNc MPO dans le plasmide de tranfert pour Baculovirus

L'ADN du plasmide de transfert pAcYMI (Baculovirus (réf. : Matsuura et al. J. Gen. Virol (1987) 68, 1233-1250 a été linéarisé par BamHI et mélangé au fragment d'ADN HpaI-HindIII de 2261 pb correspondant à la MPO. Le mélange a été traité à la T4 ADN polymérase, ligué et utilisé pour transformer des cellules compétentes E. coli MM294. La sélection des clones a été faite par croissance sur ampicilline. Après plusieurs restrictions enzymatiques contôles sur 48 clones, le clone 11 a été identifié pour avoir l'insert MPO dans la bonne orientation par rapport au promoteur de la polyhédrine (pNIV2704) (figure 5).
Les jonctions oligonucléotides synthétiques/MPO additionnées en 5' et en 3' au cDNA MPO lors des constructions précédentes ont été confirmées par séquençage de ces régions. La méthode de séquençage sur ADN double brin avec la séquenase a été utilisée.

"Co-transfection" et "plaque assay"

Le plasmide recombinant 11 a été utilisé en conjonction avec l'ADN viral sauvage pour co-transfecter des cellules *Spodoptera frugiperda* (Sf9) en culture (Le protocole est bien connu et est détaillé dans le manuel de M.D. Summers et G.E. Smith. "A Manual of Methods for Baculovirus Vectors and Insect Cell Culture Procedures, Texas University, College Station. 1987).
Les surnageants de cotransfection de jours 5 et 7. après recombinaison homologue, ont été utilisés en "plaque assay" de manière à avoir 100 à 1000 plages de lyse par boîte. Les virus recombinants ne contenant pas de polyhédrine ont été identifiés 5 jours plus tard visuellement ou par hybridation d'ADN. 13 candidats ont été repiqués et purifiés.

Test de production de MPO recombinante

Deux Baculovirus recombinants, dénommés MPO1.1 et MPO5.2 ont été utilisés pour infecter des cellules Sf9 et mesurer la capacité de celles-ci de produire de la myéloperoxydase. Dans les deux cas, les cellules infectées secrètent dans le milieu de culture une protéine reconnue spécifiquement par les anticorps anti myéloperoxydase et dont la quantité a été évaluée par test ELISA à ± 0,2 $\mu$g/ml. (Les cellules infectées ont été récoltées à la densité de $10^6$ cellules/ml). Le produit recombinant a été retrouvé aussi dans l'extrait cellulaire brut à raison de ± 0,06 $\mu$g/ 10° cellules.

. Co-transfection des cellules d' insectes Sf9 (*Spodoptera frugiperda*). Utilisation du vecteur recombinant construit (pNIV2704) PAcYM1/MPO 11 contenant la MPO en aval du promoteur baculovirus de la polyhédrine, 100 $\mu$g utilisés en conjonction avec 1 $\mu$g d'ADN viral.
Technique de transfection au chlorure de calcium.
. Utilisation du surnageant de 5 j de co-transfection pour le "plaque assay". Dilutions de -1 à -7; 5 boîtes par dilution.
. Utilisation du surnageant de 7j, dans les mêmes conditions.

Les surnageants récoltés ont été utilisés pour produire des plages de lyse dans des monocouches de cellules Sf9 (infection des cellules - 3 millions par boîte - par des dilutions du surnageant de co-transfection. pendant 60'. Enlever l' inoculum et couler une couche d' agar à bas point de fusion l,5% final. Recouvrir de milieu EX-Cell 400 J.R.Scientific additionné d'antibiotiques et laisser à l'étuve à 28°C pendant 4 jours). Coloration des boîtes au rouge neutre après 4 j d'infection et hybridation des filtres avec un fragment d'ADN MPO marqué au $\alpha^{32}P$ dCTP Repiquages de 13 candidats d'après l'autoradiographie.
Deux "plaque assay purification" ont été réalisés ensuite successivement.

D. Clonage de l'ADNc hMPO dans le vecteur d'expression pTDN pour cellules de mammifères (CHO) et introduction dans les cellules.

1. Protocole

a- Obtention du vecteur d'expression pNIV2703 :

Dans le but de faire produire la hMPO par des cellules ovariennes de hamster chinois (CHO), nous avons introduit la cassette HindIII/SnaBI de 2253 pb du pNIV2702 portant les séquences codant pour la hMPO entre les sites HindIII et SnaBI du vecteur d'expression mammifère pTDN. Le vecteur pTDN, dont le principe d'utilisation est identique au vecteur pTND (Connors et al., 1988) dont il est issu, porte 2 gènes encodant des marqueurs de sélection [résistance à la néomycine (néophosphotransférase bactérienne, $Neo^R$) et dihydrofolate réductase (DHFR)] et une cassette d'expression de la molécule d'intérêt, dans ce cas, le ht-PA (activateur tissulaire du plasminogène humain). La manipulation décrite précédemment consiste donc à remplacer la cassette HindIII/SnaBI portant l'entièreté des séquences codant pour le ht-PA par celle encodant la hMPO. Ce remplacement une fois effectue, le plasmide recombinant obtenu, pNIV2703, a été introduit dans la souche d'E.coli MM294 dans le but d'en purifier une quantité suffisante pour la transfection des cellules de CHO. La fig. 6 décrit schématiquement le vecteur d'expression pTND (Connors et al., 1988). Le vecteur pTDN correspond au vecteur pTND excepté le fait que la cassette DHFR a un ordre de lecture inverse et est localisée entre la cassette néo et la cassette t-PA.

b- Transfection par électroporation des cellules CHO :

Grâce à une digestion par l'enzyme de restriction Not 1. les sequences d'origine bactérienne (PUC19 sur la fig. 6 ont été séparées du fragment portant les 3 cassettes d'expression pour cellules de mammifères. Une fois digéré, le vecteur pNIV2703 est introduit par électroporation, une méthode de transfection, dans des cellules de CHO DHFR suivant une méthode analogue à celle décrite par Zerbib et al. (1985, Biochem. Biophys. Res. Comm. 129 , 611-618). Les cellules ont ensuite été placées dans un milieu de croissance contenant du G418, lequel ne permet pas aux cellules n'exprimant pas la néophosphotransférase de survivre. Ainsi, après une période de 1 à 3 semaines, seules les cellules ayant acquis le gêne de sélection adéquat porté par le vecteur pNIV2703 survivent et se multiplient. Les clones de cellules ainsi obtenus ont finalement été testés pour l'expression de la hMPO. Pour cela, le surnageant de culture et un extrait cellulaire de chacun de ces clones a été analysé grâce à un test ELISA (Enzyme Linked Immunosorbent Assay) permettant de qualifier et quantifier spécifiquement la hMPO.

Les résultats indiquent que les clones recombinants secrètenL dans le milieu de culture de la MPO rocombinante. Le taux de production, estimé Par ELISA, est compris entra 0. 1 et 1 microgramme par ml de surnageant.

Exemple 2 : Application thérapeutique de la hMPO Rôle de la myéloperoxydase leucocytaire

La défense de notre organisme contre des microorganismes étrangers est assurée par les globules blancs ou leucocytes parmi lesquels on trouve les lymphocytes qui produisent des anticorps, les macrophages, les éosinophiles et les neutrophiles (ou polymorphonucléaires) qui détruisent le microorganisme étranger par phagocytose. Au cours de celle-ci les neutrophiles génèrent des espèces oxygénées très toxiques et bactéricides : l'anion superoxyde, le peroxyde d'hydrogène, le radical hydroxyle, l'oxygène singulet.

L'action bactéricide du peroxyde d'hydrogène ($H_2O_2$) est augmentée de mille fois par la myéloperoxydase (MPO), enzyme localisée dans les granules azurophiles (ou primaires) des neutrophiles. En effet, cette enzyme catalyse, en présence d'$H_2O_2$, l'oxydation d'ion chlorure ($Cl^-$) pour générer de l'acide hypochloreux (HOCl) (1) qui présente des propriétés bactéricides.

$$H_2O_2 + Cl^- \xrightarrow[H^+]{MPO} HOCl + H_2O$$

Les monocytes qui sont les précurseurs des macrophages possèdent un mécanisme d'activité antimicrobienne semblable aux neutrophiles mais ils ne produisent que peu d'$H_2O_2$ et de plus ils possèdent

environ trois fois moins de myéloperoxydase que les neutrophiles (2). D'autre part, des expériences in vitro ont montré que, au cours de leur maturation en macrophages, ils perdent totalement leur contenu en myéloperoxydase, ce qui se traduit également par une décroissance de l'activité antibactérienne (3). Ainsi des monocytes fraîchement isolés ont-ils une cytotoxicité de 90 % vis-à-vis de Toxoplasmes gondii ingérés tandis que les macrophages (10 jours de culture) ne présentent plus que 12 % de cytotoxicité.

Cependant plusieurs travaux ont montré que les macrophages pouvaient phagocyter des débris cellulaires de neutrophiles (4, 5) et acquérir ainsi une activité myéloperoxydasique, avec comme conséquence une augmentation de la toxicité de la faible quantité de peroxyde d'hydrogène produite par les macrophages. D'autres études ont souligné l'importance de l'augmentation de l'activité poroxydasique des macrophages puisque, si les T gondii sont préalablement incubés avec de la peroxydase d'éosinophiles de chevaux, les macrophages retrouvent alors une cytotoxicité de 90 %, équivalente à celle des monocytes (3, 6). Les mêmes observations ont été faites avec S. aureus (7),le T. cruzi (8) ou des cellules tumorales (9).

## MATERIELS ET METHODES

### Purification des monocytes humains.

50 ml de sang d'un sujet normal sont prélevés sur Calciparine (0,3 ml à 25 000 U/ml) puis dilués de moitié avec du tampon PBS 0,01M pH 7,2. 35 ml de sang dilué sont alors déposés un gradient de densité constitué de 15 ml de Ficoll-Paque (Pharmacia).

Après une centrifugation à température ambiante à 1800 t/min pendant 30 minutes, les lymphocytes et les monocytes situés à l'interface du gradient sont récupérés et conservés sur glace. Les cellules sont ensuite lavées une première fois avec du tampon PBS puis centrifugées à 2000 t/min pendant 10 minutes à 4 ° C. L'opération est encore répétée deux fois puis les cellules sont mises en culture.

### Culture des monocytes humains.

Les monocytes sont purifiés par adhérence sur verre. Dans des alvéoles d'un diamètre de 2,5 cm (Limbro), les cellules sont mises en contact avec 1 ml de milieu de culture MEM contenant 10 % de sérum humain. Après une incubation à 37 ° C pendant 2 heures dans une atmosphère à 5 % $CO_2$, les cellules non-adhérentes (lymphocytes) sont éliminées en enlevant le surnageant. Les monocytes adhérents sont remis en contact avec 1 ml de MEM contenant également 10 % de sérum humain puis laissés en culture.

## I. Mise en évidence par microscopie de l'incorporation de la myéloperoxydase dans les monocytes

### I.1 Méthode

Les monocytes sont mis en présence de 980 ng/ml de myéloperoxydase leucocytaire humaine semipurifiée. Après 4 heures d'incubation à 37 ° C,le milieu de culture MEM est enlevé et les monocytes sont lavés soigneusement avec du tampon PBS. Les monocytes sont ensuite décrochés par agitation forte avec 1 ml de tampon PBS puis centrifugés de façon à former un dépôt sur une lame de microscope.

Les lames sont fixées par une solution d'éthanol-formaldéhyde (9:1) à température ambiante, lavées ensuite avec de l'eau puis séchées à l'air. L'activité peroxydasique des monocytes est mise en évidence en déposant sur la lame quelques gouttes d'un mélange de benzidine 1 % (30 ml), de nitroprvssiate de sodium 4% dilué au dixième (0,3 ml) et 0,3 ml d'$H_2O_2$ dilué 80 fois.

Après un contact de 2 minutes à température ambiante, les lames sont lavées abondamment avec de l'eau puis séchées à l'air.

Dans une dernière étape, les lames sont colorées avec du Giemsa pour la mise en évidence des cellules.

Les lames sont ensuite visualisées au microscope.

### 1.2 Résultats

Des monocytes témoins ne présentent que très peu de réaction positive à la benzidine. Seuls quelques monocytes développent une coloration légèrement brunâtre, démontrant bien ainsi que la myéloperoxydase n'est présente qu'en faible quantité dans ces cellules.

Par contre, des monocytes en culture depuis 24 heures et mis en contact avec de la myéloperoxydase réagissent positivement et de manière très nette à la benzidine . Ces premiers résultats confirment une

incorporation de la myéloperoxydase par simple phagocytose dans les monocytes.

II. Mise en évidence par radioimmunoessai de l'incorporation de la myéloperoxydase par les monocytes

II.1 Méthode

Après une mise en contact des monocytes adhérents avec 1 ml de tampon MOM contenant de la myéloperoxydase semi-purifiée ou des débris de neutrophiles obtenus par sonication (le protocole de quatre expériences est décrit en détail dans les résultats), le surnageant est récupéré puis centrifugé à 2000 t/min pendant 10 minutes afin de récupérer les monocytes qui se seraient éventuellement décrochés (culot 1).

Les monocytes adhérents sont décrochés par agitation efficace avec 1 ml de tampon PBS. Ils sont ajoutés au culot 1 puis centrifugés à 2000 t/min pendant 10 minutes à 4 °C. Après trois lavages avec du tampon PBS, les cellules sont comptées puis diluées afin d'obtenir 3 millions de monocytes par ml.

La myéloperoxydase des monocytes est solubilisée en traitant les cellules avec du bromure de cetyltriméthylammonium (0,01 %) et par deux congélations successives.

Après le retour à température ordinaire, 100 µl du milieu sont prélevés pour la quantification de l'enzyme selon une technique de radioimmunoessai spécifique (10).

II.2 Résultats

Expérience 1

Trois millions de monocytes en culture depuis 24 heures sont mis en contact pendant 2 heures avec 1 ml de milieu de culture MEM contenant :

a) MPO 1 = myéloperoxydase semi-purifiée à une concentration de 980 ng/ml.
b) MPO 2 = 50 µl d'une suspension concentrée de débris de neutrophiles soniqués.

| 1 | myélo-peroxy-dase ng/ml | % augmen-tation du contenu intracel-lulaire |
|---|---|---|
| Monocytes 24h (3 millions) | 80 | |
| Monocytes 24h + MPO 1 } 2 heures incubation | 88 | 1,1 |
| Monocytes 24h + MPO 2 } 2 heures incubation | 358 | 447 |

Le taux basal de monocytes n'ayant pas été mis en contact avec de la myéloperoxydase est de 80 ng/ml. Après une incubation de 2 heures avec de la myéloperoxydase semi-purifiée, le taux intracellulaire en myéloperoxydase des monocytes reste inchangé. Par contre, une forte augmentation (447 % du taux basal) de la concentration intracellulaire en myéloperoxydase est observée lorsque les monocytes sont mis au contact de débris de neutrophiles. Cette dernière observation montre que les monocytes sont bien capables de phagocyter des débris de neutrophiles.

Expérience 2

Les conditions sont identiques que dans l'expérience 1, excepté que le temps d'incubation est de 4 heures. On constate cette fois une nette augmentation (440 %) du contenu intracellulaire en myéloperoxydase des monocytes ayant été mis en contact avec l'enzyme. De même, les débris de neutrophiles sont

encore mieux phagocyter que dans l'expérience précédente puisqu'il y a une augmentation de 2070 % du contenu intracellulaire en myéloperoxydase.

| 2 | myélo-peroxy-dase ng/ml | % augmen-tation du contenu intracel-lulaire |
|---|---|---|
| Monocytes 24h (3 millions) | 54 | |
| Monocytes 24h + MPO 1 } 4 heures incubation | 238 | 440 |
| Monocytes 24h + MPO 2 } 4 heures incubation | 1120 | 2070 |

Expérience 3

Le tableau ci-dessous montre que des monocytes en culture depuis 48 heures incorporent mieux la myéloperoxydase ou les débris de neutrophiles après 2 heures d'incubation que des monocytes en culture depuis 24 heures.

| 3 | myélo-peroxy-dase ng/ml | % augmen-tation du contenu intracel-lulaire |
|---|---|---|
| Monocytes 48h (3 millions) | 102 | |
| Monocytes 48h + MPO 1 } 2 heures incubation | 204 | 220 |
| Monocytes 48h + MPO 2 } 2 heures incubation | 788 | 772 |

Expérience 4

Des monocytes en culture depuis 24 heures sont mis cette fois en contact pendant 2 heures et 6 heures avec 1 ml de tampon de culture contenant 3920 ng/ml de myéloperoxydase semi-purifiée (MPO3).

| 4 | myéloperoxydase | | % augmentation du contenu cellulaire |
|---|---|---|---|
| | ng/ml | U/ml | |
| Monocytes 24h (3 millions) | 90 | 0,6 | |
| Monocytes 24h + MPO 3 (2 heures incubation) | 136 | 0,86 | 151 |
| Monocytes 24h + MPO 3 (6 heures incubation) | 324 | 1,3 | 360 |

Après 2 heures de mise en contact avec l'enzyme, les monocytes augmentent leur contenu intracellulaire de 151 % alors que dans l'expérience 1, ce contenu était resté inchangé. L'augmentation est encore plus nette après 6 heures de mise en contact. Dans cette expérience. on a également mesuré dans les monocytes l'activité enzymatique de la myéloperoxydase déterminée par l'oxydation de l'o-dianisidino en présence d'$H_2O_2$. Le taux basal de 0,6 U/ml augmente au fur et à mesure que le temps de mise en contact augmente (valeur doublée après 6 heures). Cette constatation est la preuve que la myéloperoxydase exogène incorporée dans le monocyte reste bien enzymatiquement active.

III. Cytotoxicité des monocytes ayant incorporé de la myéloperoxydase vis-à-vis de schistosomules

III.1 Méthode

Les schistosomules sont isolés à partir de cercaires par une technique artificielle (filtration à travers un morceau de peau de souris). Le protocole expérimental suivant a été retenu : des monocytes de 24 heures sont incubés pendant 2 heures avec de la myéloperoxydase puis lavés soigneusement avec du milieu de culture MEM. Ensuite, les monocytes traités ou non avec de la myéloperoxydase sont incubés pendant 6 heures avec du sérum d'un sujet atteint de bilharziose (10 %) ou avec un sérum d'un sujet sain (10 %) qui sert de contrôle (effet propre du sérum). Des schistosomules préalablement traités ou non avec de la myéloperoxydase sont ensuite ajoutés au milieu de culture. Après 16 heures, les schistosomules vivants et morts sont comptés et un % de cytotoxicité des monocytes est ainsi déterminé.
Monocytes : 100 à 200 000 cellules en culture depuis 24 heures

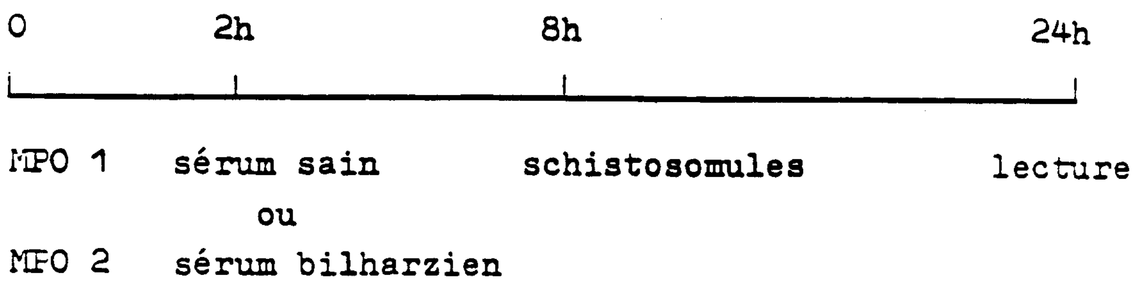


Expérience 5

Ces résultats montrent que la cytotoxicité des monocytes ayant préalablement incorporés MPO 1 ou MPO 2 vis-à-vis des schistosomules en présence de sérum de bilharzien est augmentée de 50 % par rapport aux monocytes témoins. Le pourcentage réel de cytotoxicité est obtenu en soustrayant la valeur obtenue avec le sérum sain (effet propre du sérum) de la valeur obtenue avec le sérum de bilharzien.

| 5 | % cytotoxicité des monocytes | | |
|---|---|---|---|
| | sans MPO | + MPO 1 | + MPO 2 |
| sérum bilharzien 10 % | 42,5 ± 5 | 70,5 ± 10,5 | 71,5 ± 3 |
| sérum sain 10 % | 19 ± 0 | 26 ± 1,4 | 25 + 5 |
| % réel cytotoxicité | 23,5 | 44,5 | 46,5 |

Expérience 6

Dans cette expérience, on a comparé la cytotoxicité de monocytes ayant ou n'ayant pas incorporé de la myéloperoxydase Vis-à-vis de schistosomules normaux (stimulus 1) et de schistosomules ayant été préalablement mis en contact pendant 2 heures avec 980 ng/ml de myéloperoxydase (stimulus 2).

| 6 | % cytotoxicité des monocytes | | | |
|---|---|---|---|---|
| | sans | MPO | + MPO | 1 |
| | stimulus 1 | stimulus 2 | stimulus 1 | stimulus 2 |
| sérum bilharzien 10 %<br>sérum sain 10 % | 42,5 ± 5<br>19 ± 0 | 62,5 ± 2<br>22 ± 4 | 70,5±10,5<br>26 ± 1,4 | 99<br>35,5 ± 2 |
| % réel cytotoxicité | 23,5 | 40,5 | 44,5 | 64 |

Les monocytes non traités avec de la myéloperoxydase ont une cytotoxicité qui passe de 23,5 à 40,5 % lorsqu'ils sont mis en contact avec des schistosomules recouverts de myéloperoxydase.

Bien que le modèle soit différent (dans ce cas, le monocyte ne tue pas le schistosomule par phagocytose mais par simple adhérence avec lui), cette observation rejoint les travaux antérieurs montrant que la cytotoxicité des macrophages est augmentée lorsque l'organisme infectieux (T. gondii) phagocyté est recouvert avec une peroxydase (3,6).

La combinaison de monocytes ayant incorporé de la myéloperoxydase avec des schistosomules recouverts avec l'enzyme permet d'obtenir une cytotoxicité très élevée (64 %).

Conclusions

Il était seulement montré dans la littérature que les monocytes peuvent acquérir une peroxydase accompagnée, c'est-à-dire associée à un support qui est un microorganisme et voir ainsi leur cytotoxicité augmentée vis-à-vis de toute une série d'organismes infectieux. Dans ces expériences, il faut en effet remarquer que la myéloperoxydase était d'abord liée à l'organisme infectieux qui était ensuite ingéré par le monocyte ou macrophage.

Selon l'invention il a été utilisé de la myéloperoxydase granulocytaire humaine et on a découvert que :

1°) la myéloperoxydase peut être directement phagocytée sans l'intervention d'un activateur par le monocyte,

2°) l'enzyme ingérée reste enzymatiquement très active ainsi que le montrent les résultats de cytotoxicité vis-à-vis des schistosomules.

Ces observations indiquent que de la myéloperoxydase exogène administrée dans l'organisme sera reprise par les monocytes ou macrophages humains et dès lors peut être utilisée comme un moyen de thérapeutique chez des patients souffrant de déficiences héréditaires (agranulocytose) ou acquise (SIDA).

Références


1. Zgliczynski JM, Stelmaseynska T, Domanski J and Ostrowski W. Chloramines as intermediates of oxidative reaction of amino acids by myeloperoxidase. Biochim Biophys Acta 1971, 235:419-424.
2. Dos A, Wever R and Roos D. Characterization and quantification of the peroxidase in human monocytes. Biochim Biophys Acta 1978, 525:37-44.
3. Locksley RM, Nelson CS, Fankhauser JE and Klebanoff SJ. Loss of granule myeloperoxidase during in vitro culture of human monocytes correlates with decay in antiprotozoa activity. Am J Trop Med Hyg 1987, 36(3):541-548.
4. Hoifets L, Katsuyuki I and Mayer G. Expression of peroxidase-dependent iodination by macrophages ingesting neutrophils debris. J. Reticuloendothel Soc 1980, 28(3): 391-404.
5. Atwal O. Cytoenzymological bohavior of peritoneal exudate cells of rat in vivo. J.Reticuloendothel Soc 1971, 10:163-172.
6. Locksley RM, Wilson CB and Klebanoff SJ. Role of endogenous and acquired peroxidase in the toxoplasmacidal activity of murine and human mononuclear phagocytes. J. Clin Invost 1982, 69:1099-1111.

7. Ramsey PG, Martin T, Chi E and Klebanoff SJ. Arming of mononuclear phagocytes by eosinophil peroxidase bound to staphylococcus aureus. J. Immunol 1982, 128:415-420.
8. Nogueira NH, Klebanoff SJ and Cohn ZA. Teruzi : sensitization to nacrophage killing by eosinophil peroxidase. J Immunol 1982, 128:1705-1708.
9. Nathan CF and Klebanoff SJ. Augmentation of spontaneous macrophage-mediated cytolysis of eosinophil peroxidase. J Exp Med 1982, 155:1291 -1308.
10.Deby-Dupont G, Pincemail J, Thirion A and Deby C. A radioimmunoassay for polymorphonuclear leucocytes myeloperoxidase : preliminary results. Arch Int Physiol Biochim 1987 (in press).

## Revendications

**1.** Myéloperoxydase humaine biologiquement active produite par culture de cellules eukaryotes transformées par un vecteur d'expression de la hMPO dans lesdites cellules, à l'exclusion de l'enzyme naturelle.

**2.** Myéloperoxydase humaine selon la revendication 1, caractérisée en ce qu'elle est produite par une culture de cellules d'insectes ou de mammifères.

**3.** Cassette d'expression de la myéloperoxydase humaine consistant dans le fragment Hind III/SnaBI, HindIII/EcorV ou HindIII/Hpal portant la séquence codante de la hMPO telle que représentée sur la figure 1.

**4.** Plasmide vecteur de clonage contenant une cassette selon la revendication 3.

**5.** Vecteur d'expression dans des cellules eukaryotes comportant une cassette selon la revendication 3.

**6.** Plasmide de transfert recombinant pour Baculovirus contenant l'ADNc de la hMPO sous le contrôle du promoteur de polyhédrine.

**7.** Plasmide pNIV2704 tel que représenté à la figure 5.

**8.** Cellules d'insectes co-transfectées par un plasmide selon les revendications 6 et 7 et de l'ADN viral sauvage.

**9.** Cellules d'insectes de spodoptera frugiperda infectées par un Baculovirus recombinant obtenu à partir d'un plasmide selon les revendications 6 et 7.

**10.** hMPO produite par culture de cellules d'insectes, spodoptera frugiperda transfectées par un vecteur d'expression dans lesdites cellules selon la revendication 9.

**11.** Vecteur d'expression de la hMPO dans des cellules de CHO contenant la cassette HindIII/SnaBI portant la séquence codante totale pour la hMPO telle que représentée sur la figure 1.

**12.** Plasmide selon la revendication 11 consistant dans le plasmide pNIV2703 caractérisé en ce qu'il comporte la cassette HindIII/SnaBI portant le gène codant la hMPO inséré dans un vecteur pTND en remplaçant, tel que le décrit la figure 6, la cassette HindIII/SnaBI portant la séquence codant pour le ht-PA par celle codant la hMPO.

**13.** hMPO produite par culture de cellules de mammifères, notamment de cellules CHO transfectées par un vecteur selon les revendications 11 et 12.

**14.** Médicament consistant en la hMPO recombinante selon l'une des revendications 1, 2, 10 et 13.

**15.** Médicament consistant en un composé chimique constitué par l'enzyme myelopéroxydase humaine conjuguée.

**16.** Médicament selon la revendication 14, caractérisé en ce que la myelopéroxydase est conjuguée par couplage covalent ou complexation à un transporteur présentant une affinité pour les macrophages.

**17.** Médicament selon la revendication 15, caractérisé en ce que le transporteur est l'albumine humaine mannosylée ou un anticorps ou fragment d'anticorps tel que la partie constante Fc dirigée contre des récepteurs présents sur les macrophages.

**18.** Médicament selon la revendication 14, caractérisé en ce que la myelopéroxydase est encapsulée dans des liposomes.

**19.** Médicament selon l'une des revendications 14 à 17, caractérisé en ce que la myelopéroxydase est préparée par la technique de l'ADN recombinant selon l'une des revendications 1 à 13.

**20.** Médicament selon l'une des revendications 15 à 19 utile pour lutter contre les infections à l'intérieur des macrophages.

**21.** Utilisation de la myelopéroxydase humaine libre ou du médicament selon l'une des revendications 15 à 20 pour la préparation de compositions pharmaceutiques utiles pour lutter contre les infections à l'intérieur des macrophages.

**22.** Utilisation de la myelopéroxydase humaine libre ou du médicament selon l'une des revendications 15 à 21 pour la préparation de compositions pharmaceutiques utiles notamment pour le traitement des immunodéficiences occasionnées notamment par le SIDA, les brûlures et les irradiations.

**23.** Composition pharmaceutique comprenant à titre de principe actif un médicament selon l'une des revendications 15 à 21 et un support pharmaceutiquement acceptable.

**24.** Composition pharmaceutique selon la revendication 23 utile pour le traitement des immunodéficiences occasionnées notamment par le SIDA, les brûlures ou les irradiations.

**Claims**

**1.** Biologically active human myeloperoxidase produced by culturing eukaryotic cells transformed by a vector for the expression of hMPO in the said cells, excluding the natural enzyme.

**2.** Human myeloperoxidase according to Claim 1, characterized in that it is produced by a culture of insect or mammalian cells.

**3.** Cassette for the expression of human myeloperoxidase, consisting of the HindIII-SnaBI, HindIII-EcoRV or HindIII-HpaI fragment carrying the coding sequence for hMPO as shown in Figure 1.

**4.** Plasmid cloning vector containing a cassette according to Claim 3.

**5.** Vector for expression in eukaryotic cells, containing a cassette according to Claim 3.

**6.** Recombinant transfer plasmid for Baculovirus, containing the cDNA for hMPO under the control of the polyhedrin promoter.

**7.** Plasmid pNIV2704 as shown in Figure 5.

**8.** Insect cells, cotransfected by a plasmid according to Claims 6 and 7 and wild-type viral DNA.

**9.** Insect cells of Spodoptera frugiperda infected by a recombinant Baculovirus obtained from a plasmid according to Claims 6 and 7.

**10.** hMPO produced by culturing Spodoptera frugiperda insect cells, transfected by a vector for expression in the said cells according to Claim 9.

**11.** Vector for the expression of hMPO in CHO cells, containing the HindIII-SnaBI cassette carrying the complete coding sequence for hMPO as shown in Figure 1.

12. Plasmid according to Claim 11, consisting of plasmid pNIV2703, characterized in that it contains the HindIII-SnaBI cassette carrying the gene encoding hMPO inserted into a pTND vector by replacing, as depicted in Figure 6, the HindIII-SnaBI cassette carrying the sequence coding for ht-PA by that encoding hMPO.

13. hMPO produced by culturing mammalian cells, in particular CHO cells, transfected by a vector according to Claims 11 and 12.

14. Medicinal product consisting of recombinant hMPO according to one of Claims 1, 2, 10 and 13.

15. Medicinal product consisting of a chemical compound consisting of the enzyme human myeloperoxidase in conjugated form.

16. Medicinal product according to Claim 14, characterized in that the myeloperoxidase is conjugated by covalent coupling or complexing to a transporting agent possessing an affinity for macrophages.

17. Medicinal product according to Claim 15, characterized in that the transporting agent is mannosylated human albumin or an antibody or antibody fragment, such as the Fc constant portion, directed against receptors present on macrophages.

18. Medicinal product according to Claim 14, characterized in that the myeloperoxidase is encapsulated in liposomes.

19. Medicinal product according to one of Claims 14 to 17, characterized in that the myeloperoxidase is prepared by the recombinant DNA technique according to one of Claims 1 to 13.

20. Medicinal product according to one of Claims 15 to 19, which is useful for combating infections within macrophages.

21. Use of free human myeloperoxidase or of the medicinal product according to one of Claims 15 to 20 for the preparation of pharmaceutical compositions useful for combating infections within macrophages.

22. Use of free human myeloperoxidase or of the medicinal product according to one of Claims 15 to 21 for the preparation of pharmaceutical compositions useful, in particular, for the treatment of immunodeficiencies caused, in particular, by AIDS, burns and irradiation.

23. Pharmaceutical composition comprising, by way of active principle, a medicinal product according to one of Claims 15 to 21 and a pharmaceutically acceptable vehicle.

24. Pharmaceutical composition according to Claim 23, which is useful for the treatment of immunodeficiencies caused, in particular, by AIDS, burns or irradiation.

**Patentansprüche**

1. Biologisch aktive Human-Myeloperoxidase, hergestellt durch Kultivierung von Eukarioten-Zellen, die durch einen Expressionsvektor der hMPO in den genannten Zellen transformiert worden sind, unter Ausschluß des natürlichen Enzyms.

2. Human-Myeloperoxidase nach Anspruch 1, dadurch gekennzeichnet, daß sie durch Kultivierung von Insekten- oder Säugetier-Zellen hergestellt worden ist.

3. Kassette zur Expression der Human-Myeloperoxidase, die besteht aus dem Fragment HindIII/SnaBI, HindIII/EcorV oder HindIII/HpaI, welches die für hMPO codierende Sequenz trägt, wie sie in der Fig. 1 dargestellt ist.

4. Klonierungsvektor-Plasmid, das eine Kassette nach Anspruch 3 enthält.

5. Expressionsvektor in Eukarioten-Zellen, der eine Kassette nach Anspruch 3 enthält.

**6.** Rekombinantes Transfer-Plasmid für Baculovirus, das die cDNA der hMPO unter der Kontrolle des Polyhedrin-Promotors enthält.

**7.** Plasmid pNIV2704, wie es in der Fig. 5 dargestellt ist.

**8.** Insektenzellen, die durch ein Plasmid nach den Ansprüchen 6 und 7 und die Wild-Virus-DNA kotransfektiert worden sind.

**9.** Insektenzellen von Spodoptera frugiperda, die durch ein rekombinantes Baculovirus infiziert worden sind, das aus einem Plasmid nach den Ansprüchen 6 und 7 erhalten wurde.

**10.** hMPO, hergestellt durch Kultivierung von Insektenzellen von Spodoptera frugiperda, die durch einen Expressionsvektor in den genannten Zellen nach Anspruch 9 transfektiert worden sind.

**11.** Expressionsvektor der hMPO in CHO-Zellen, der die Kassette HindIII/SnaBI enthält, welche die für hMPO codierende Gesamtsequenz trägt, wie in der Fig. 1 dargestellt.

**12.** Plasmid nach Anspruch 11, das besteht aus dem Plasmid pNIV2703, dadurch gekennzeichnet, daß es die Kassette HindIII/SnaBI enthält, die das für hMPO codierende Gen trägt, das in einen Vektor pTN inseriert worden ist, indem man, wie in der Fig. 6 dargestellt, die Kassette HindIII/SnaBI, welche die für ht-PA codierende Sequenz trägt, durch diejenige, die für hMPO codiert, ersetzt.

**13.** hMPO, hergestellt durch Kultivierung von Säugetierzellen, insbesondere CHO-Zellen, die durch einen Vektor nach den Ansprüchen 11 und 12 transfektiert worden sind.

**14.** Arzneimittel, das besteht aus rekombinanter hMPO nach einem der Ansprüche 1, 2, 10 und 13.

**15.** Arzneimittel, das besteht aus einer chemischen Verbindung, die aus dem konjugierten Human-Myeloperoxidase-Enzym aufgebaut ist.

**16.** Arzneimittel nach Anspruch 14, dadurch gekennzeichnet, daß die Myeloperoxidase durch kovalente Kupplung oder Komplexbildung mit einem Transporteur (Träger), der eine Affinität für die Makrophagen aufweist, konjugiert ist.

**17.** Arzneimittel nach Anspruch 15, dadurch gekennzeichnet, daß der Transporteur (Träger) mannosyliertes Human-Albumin oder ein Antikörper oder ein Antikörperfragment ist, wie z.B. der konstante Teil Fc, der gegen Rezeptoren gerichtet ist, die auf den Makrophagen vorliegen.

**18.** Arzneimittel nach Anspruch 14, dadurch gekennzeichnet, daß die Myeloperoxidase in Liposome eingekapselt ist.

**19.** Arzneimittel nach einem der Ansprüche 14 bis 17, dadurch gekennzeichnet, daß die Myeloperoxidase hergestellt worden ist nach der rekombinanten DNA-Methode nach einem der Ansprüche 1 bis 13.

**20.** Arzneimittel nach einem der Ansprüche 15 bis 19, das geeignet ist für die Bekämpfung von Infektionen im Innern von Makrophagen.

**21.** Verwendung der freien Human-Myeloperoxidase oder des Medikaments nach einem der Ansprüche 15 bis 20 zur Herstellung von pharmazeutischen Zusammensetzungen für die Bekämpfung von Infektionen im Innern von Makrophagen.

**22.** Verwendung der freien Human-Myeloperoxidase oder des Arzneimittels nach einem der Ansprüche 15 bis 21 zur Herstellung von pharmazeutischen Zusammensetzungen, die insbesondere geeignet sind für die Behandlung von Immundefekten, die insbesondere verursacht worden sind durch AIDS, Verbrennungen und Bestrahlungen.

**23.** Pharmazeutische Zusammensetzung, die als Wirkstoff (aktives Prinzip) ein Arzneimittel nach einem der Ansprüche 15 bis 21 und einen pharmazeutisch akzeptablen Träger enthält.

**24.** Pharmazeutische Zusammensetzung nach Anspruch 23, die geeignet ist für die Behandlung von Immundefekten, die insbesondere durch AIDS, Verbrennungen oder Bestrahlungen hervorgerufen worden sind.

Figure 1

```
Hind III                                      Nsi I
------    Met                                 ------
 AGCTTACCATGGGGGTTCCCTTCTTCTCTTCTCTCAGATGCATGGTGGACTTAGGACCTTGCTGGGCTGGGGTCTCAC
TGCAGAGATGAAGCTGCTTCTGGCCCTAGCAGGCGTCCTGGCCATTCTGGCCACGCCCCAGCCCTCTGAAGGTGCTGCTC
CAGCTGTCCTGGGGGAGGTGGACACCTCGTTGGTGCTGAGCTCCATGGAGGAGGCCAAGCAGCTGGTGGACAAGGCCTAC
AAGGAGCGGCGGGAAAGCATCAAGCAGCGGCTTCGCAGCGGCTCAGCCAGCCCCATGGAACTCCTATCCTACTTCAAGCA
GCCGGTGGCAGCCACCAGGACGGCGGTGAGGGCCGCTGACTACCTGCACGTGGCTCTAGACCTGCTGGAGAGGAAGCTGC
                                                        Xba I
GGTCCCTGTGGCGAAGGCCATTCAATGTCACTGATGTGCTGACGCCCGCCCAGCTGAATGTGTTGTCCAAGTCAAGCGGC
TGCGCCTACCAGGACGTGGGGGTGACTTGCCCGGAGCAGGACAAATACCGCACCATCACCGGGATGTGCAACAACAGACG
CAGCCCCACGCTGGGGGCCTCCAACCGTGCCTTTGTGCGCTGGCTGCCGGCGGAGTATGAGGACGGCTTCTCTCTTCCCT
ACGGCTGGACGCCCGGGGTCAAGCGCAACGGCTTCCCGGTGGCTCTGGCTCGCGCGGTCTCCAACGAGATCGTGCGCTTC
CCCACTGATCAGCTGACTCCGGACCAGGAGCGCTCACTCATGTTCATGCAATGGGGCCAGCTGTTGGACCACGACCTCGA
CTTCACCCCTGAGCCGGCCGCCCGGGCCTCCTTCGTCACTGGCGTCAACTGCGAGACCAGCTGCGTTCAGCAGCCGCCCT
GCTTCCCGCTCAAGATCCCGCCCAATGACCCCCGCATCAAGAACCAAGCCGACTGCATCCCGTTCTTCCGCTCCTGCCCG
GCTTGCCCCGGGAGCAACATCACCATCCGCAACCAGATCAACGCGCTCACTTCCTTCGTGGACGCCAGCATGGTGTACGG
CAGCGAGGAGCCCCTGGCCAGGAACCTGCGCAACATGTCCAACCAGCTGGGGCTGCTGGCCGTCAACCAGCGCTTCCAAG
ACAACGGCCGGGCCCTGCTGCCCTTTGACAACCTGCACGATGACCCCTGTCTCCTCACCAACCGCTCAGCGCGCATCCCC
TGCTTCCTGGCAGGGGACACCCGTTCCAGTGAGATGCCCGAGCTCACCTCCATGCACACCCTCTTACTTCGGGAGCACAA
CCGGCTGGCCACAGAGCTCAAGAGCCTGAACCCTAGGTGGGATGGGGAGAGGCTCTACCAGGAAGCCCGGAAGATCGTGG
GGGCCATGGTCCAGATCATCACTTACCGGGACTACCTGCCCCTGGTGCTGGGGCCAACGGCCATGAGGAAGTACCTGCCC
ACGTACCGTTCCTACAATGACTCAGTGGACCCACGCATCGCCAACGTCTTCACCAATGCCTTCCGCTACGGCCACACCCT
CATCCAACCCTTCATGTTCCGCCTGGACAATCGGTACCAGCCCATGGAACCCAACCCCCGTGTCCCCCTCAGCAGGGTCT
TTTTTGCCTCCTGGAGGGTCGTGCTGGAAGGTGGCATTGACCCCATCCTCCGGGGCCTCATGGCCACCCCTGCCAAGCTG
AATCGTCAGAACCAAATTGCAGTGGATGAGATCCGGGAGCGATTGTTTGAGCAGGTCATGAGGATTGGGCTGGACCTGCC
TGCTCTGAACATGCAGCGCAGCAGGGACCACGGCCTCCCAGGATACAATGCCTGGAGGCGCTTCTGTGGCTCCCGCAGC
CTGAAACTGTGGGCCAGCTGGGCACGGTGCTGAGGAACCTGAAATTGGCGAGGAAACTGATGGAGCAGTATGGCACGCCC
AACAACATCGACATCTGGATGGGCGGCGTGTCCGAGCCTCTGAAGCGCAAAGGCCGCGTGGGCCCACTCCTCGCCTGCAT
CATCGGTACCCAGTTCAGGAAGCTCCGGGATGGTGATCGGTTTTGGTGGGAGAACGAGGGTGTGTTCAGCATGCAGCAGC
GACAGGCCCTGGCCCAGATCTCATTGCCCCGGATCATCTGCGACAACACAGGCATCACCACCGTGTCTAAGAACAACATC
               Bgl II
TTCATGTCCAACTCATATCCCCGGGACTTTGTCAACTGCAGTACACTTCCTGCATTGAACCTGGCTTCCTGGAGGGAAGC
                                      ------
CTCCTGATATCTACGTATGGTT                Pst I
    ***    ------ ------
   ------SnaB I  Hpa I
   EcoR V
```

Figure 2

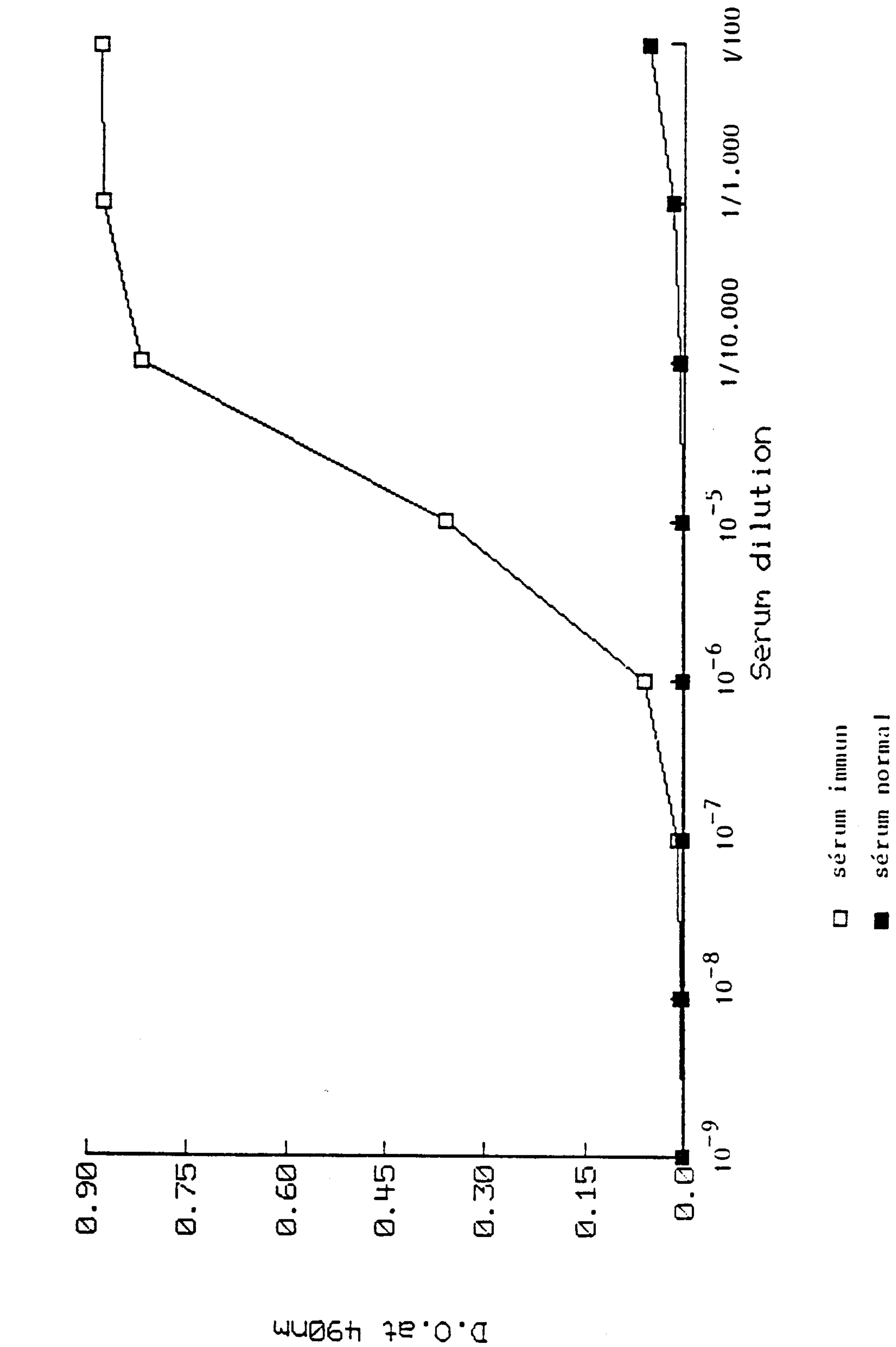
D.O.at 490nm
0.90
0.75
0.60
0.45
0.30
0.15
0.00
10^-9
10^-8
10^-7
10^-6
10^-5
1/10.000
1/1.000
1/100
Serum dilution
sérum immun
sérum normal

Figure 3

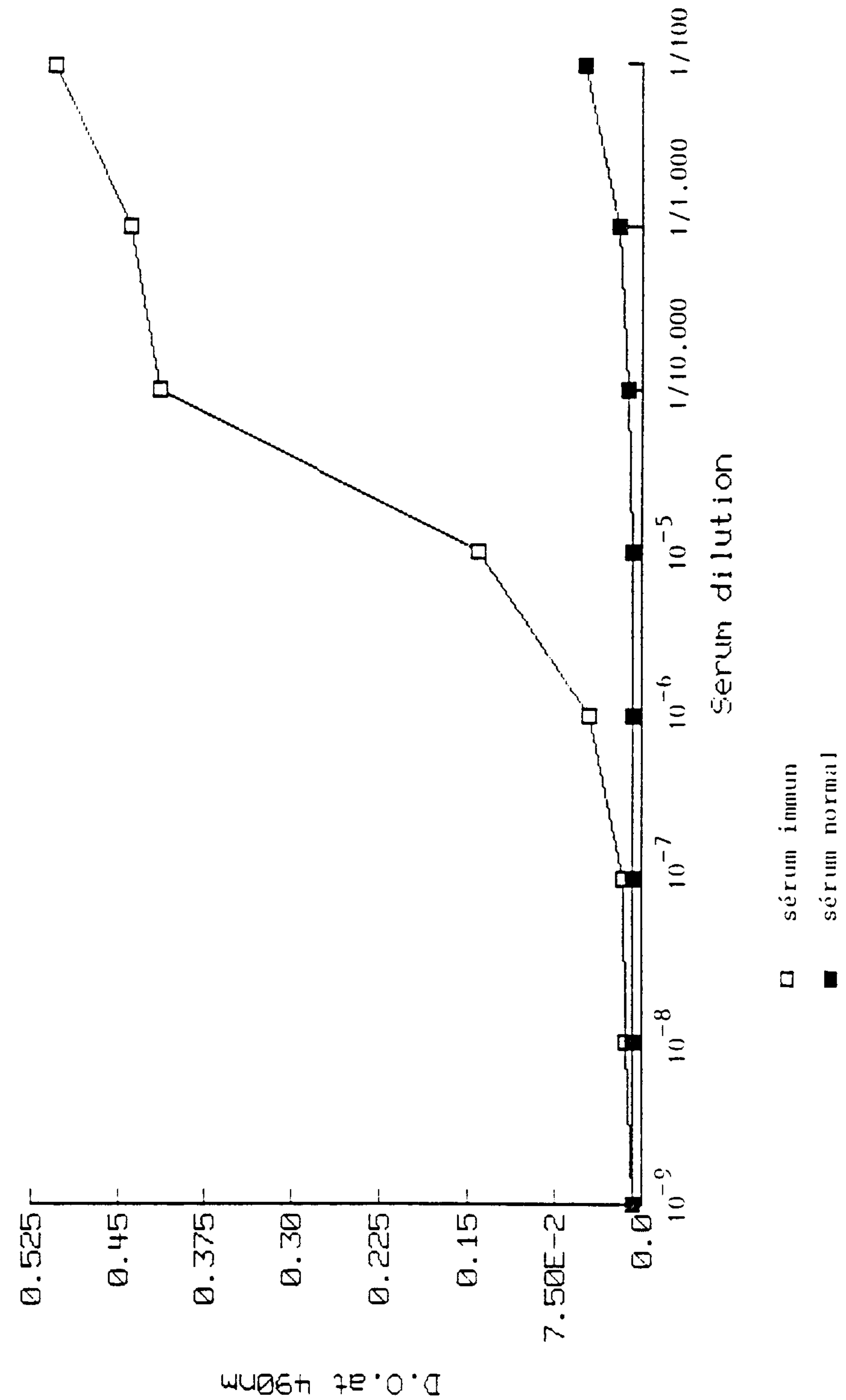
D.O. at 490nm
0.525
0.45
0.375
0.30
0.225
0.15
7.50E-2
0.0
$10^{-9}$
$10^{-8}$
$10^{-7}$
$10^{-6}$
$10^{-5}$
1/10.000
1/1.000
1/100
Serum dilution
sérum immun
sérum normal

Figure 4

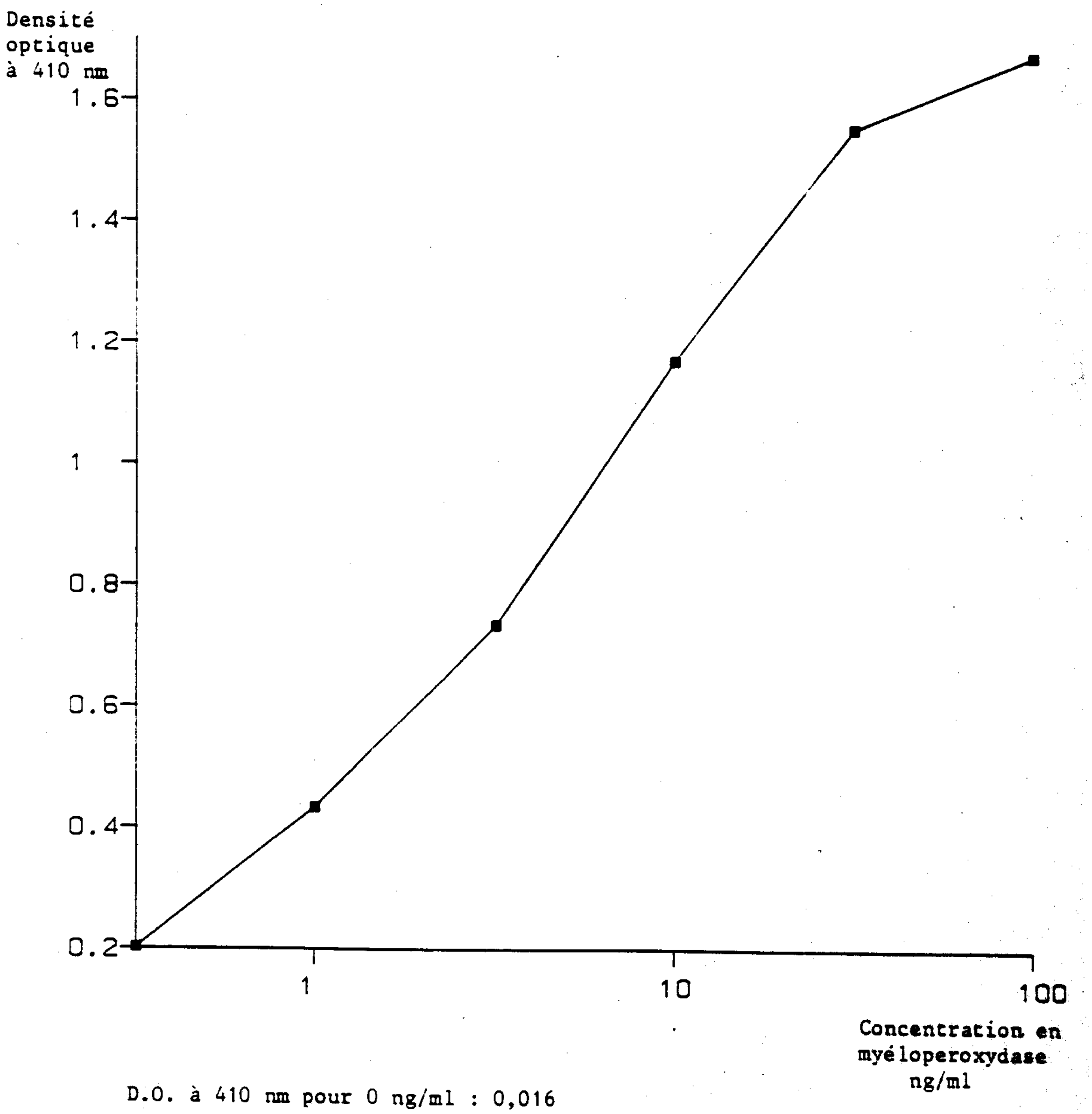
Densité
optique
à 410 nm
1.6
1.4
1.2
1
0.8
0.6
0.4
0.2
1
10
100
Concentration en
myéloperoxydase
ng/ml
D.O. à 410 nm pour 0 ng/ml : 0,016

FIG.5

Vecteur pAcYM1

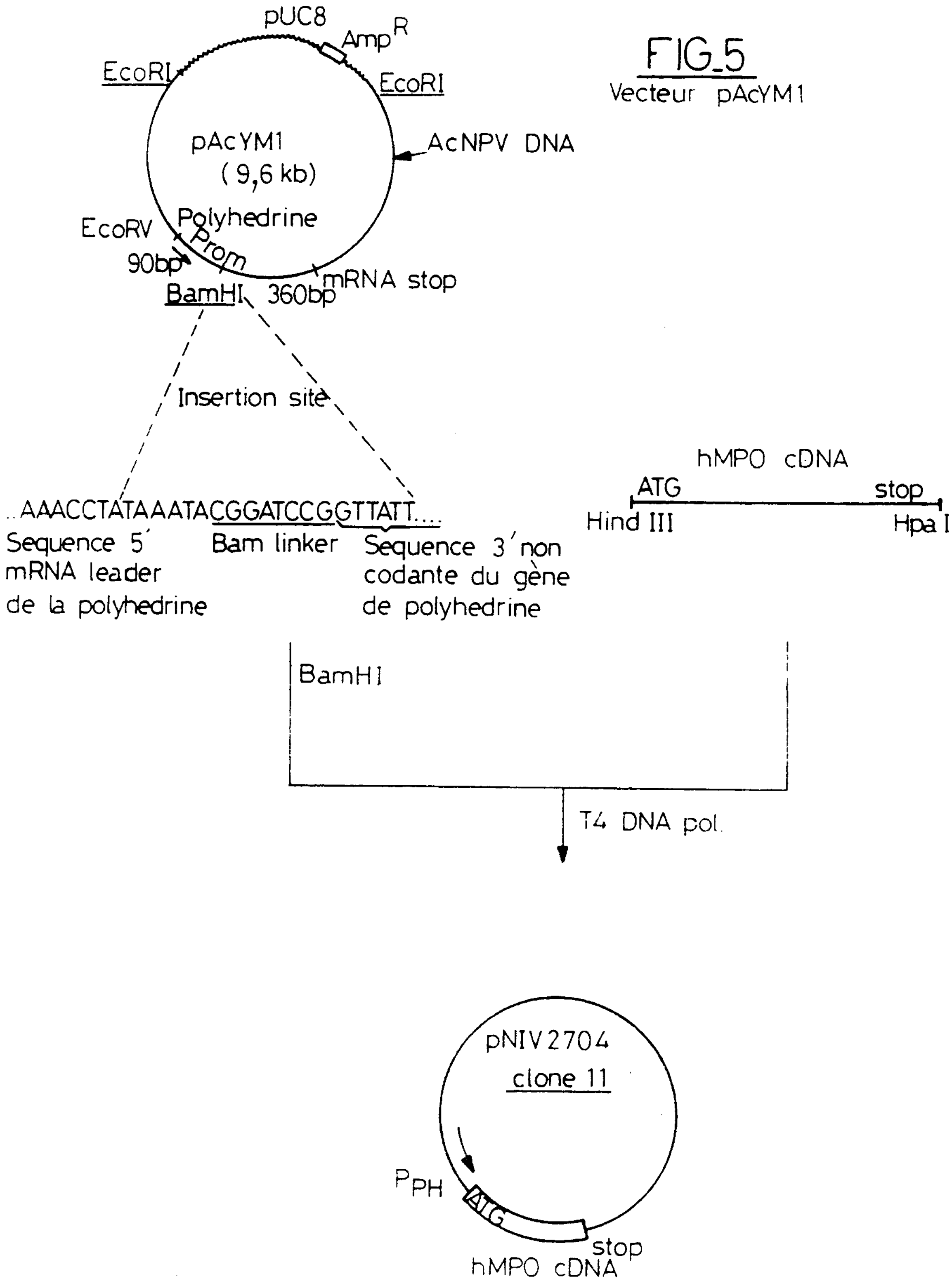

Figure 6

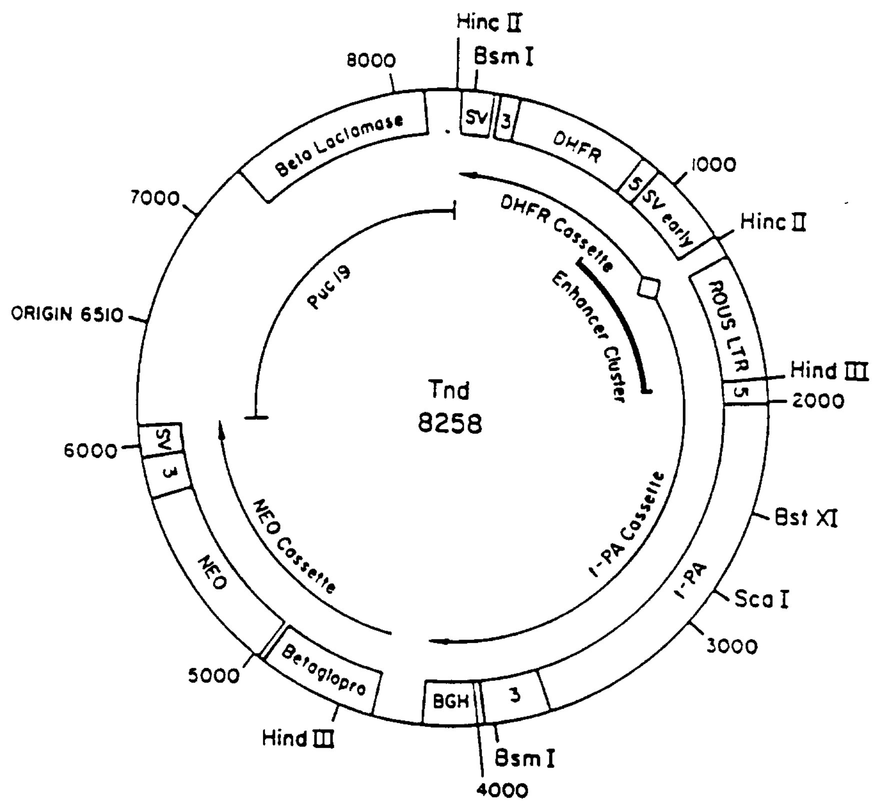
Hinc II
BsmI
8000
SV
3
DHFR
5
SV early
1000
Hinc II
ROUS LTR
Hind III
5
2000
Bst XI
t-PA
Sca I
3000
BGH
3
BsmI
4000
Hind III
Betaglopro
5000
NEO
3
SV
6000
ORIGIN 6510
7000
Beta Lactamase
DHFR Cassette
Enhancer Cluster
Puc 19
Tnd
8258
t-PA Cassette
NEO Cassette